# EUROPEAN PATENT SPECIFICATION

(11) **EP 4 329 853 B1**
(45) Date of publication and mention of the grant of the patent: **24.06.2026**
(21) Application number: 22728721.6
(22) Date of filing: 16.05.2022
(51) Int. Cl.: A61M 25/00, A61B 50/30, A61B 50/33

(54) **URINARY CATHETER-INSERTION KITS**
URINKATHETER-EINFÜHRUNGSSETS
KITS D'INSERTION DE CATHÉTER URINAIRE

(30) Priority: 26.05.2021 US 202163193440 P
(43) Date of publication of application: 06.03.2024
(73) Proprietor: C. R. Bard, Inc., Franklin Lakes, NJ 07417 (US)
(72) Inventor: CHAPMAN, Brian, Covington, GA 30014 (US)
(74) Representative: Hoffmann Eitle
(86) International application number: PCT/US2022/029394
(87) International publication number: WO 2022/250994

(56) References cited:
- WO-A1-2019/209867
- WO-A1-2019/246307
- DE-A1- 2 339 724
- US-A1- 2006 009 742

## Description

### BACKGROUND

Urinary catheters are used in healthcare facilities such as hospitals to drain urine from patients. However, catheter-associated urinary tract infections ("CAUTIs") are the second most common form of hospital-acquired infection ("HAI"). As such, hospitals are interested in ways to cut their CAUTI rates by conforming to strict aseptic techniques as part of their standard of care. But there are many factors that influence a hospital's ability to meet that standard of care. These factors include aspects of healthcare-practitioner training and experience, patient factors (e.g., general health, weight, and anatomy), environmental factors, and characteristics of urinary catheter-insertion kits including components of the kits, various layouts of the components in the kits, and instructions for use of such kits. There is a need in healthcare for an easy, safe, and reliable standard method for inserting a catheter into a patient.
WO 2019/246307 A1 relates to urinary catheter-insertion kits including a working tray, a storage tray, a urinary catheter, and a drainage system including drainage tubing and a drainage receptacle.

Disclosed herein are urinary catheter-insertion kits with integrated instructions for use and methods,which are not part of the invention, thereof.

### SUMMARY

Disclosed herein is a urinary catheter-insertion kit for a catheterization procedure. Invention is defined by claim 1 and the preferred embodiments of the invention are set forth in the dependent claims.

The urinary catheter-insertion kit includes, in some embodiments, a tray assembly, a urinary catheter, and a drainage system. The tray assembly is contoured for a space between partially opened legs of a patient. The tray assembly includes a storage tray and a working tray. The working tray includes a number of preformed sections holding a number of components of the urinary catheter-insertion kit. The working tray is suspended over the storage tray in the tray assembly, thereby forming a covered storage space in the storage tray. The urinary catheter is disposed in a catheter section of the preformed sections of the working tray. The drainage system is disposed in the storage space. The drainage system includes a drainage receptacle and drainage tubing connected to the urinary catheter.

In some embodiments, the tray assembly has a triangular prism shape.

In some embodiments, the tray assembly has a trapezoidal prism shape.

In some embodiments, the tray assembly has a flat-sided teardrop shape. Flat sides of the flat-sided teardrop shape are major sides of the tray assembly.

In some embodiments, top edges of the working tray are chamfered or filleted for patient comfort.

In some embodiments, corners between minor sides of the storage try are rounded for patient comfort.

In some embodiments, the working tray is molded plastic.

In some embodiments, the storage tray is folded paperboard.

The preformed sections of the working tray are arranged in accordance with the catheterization procedure. Earlier steps in the catheterization procedure utilize the components in the preformed sections of the working tray closer to a genital area of the patient. Later steps in the catheterization procedure utilize the components in the preformed sections of the working tray farther from the genital area.

In some embodiments, the urinary catheter-insertion kit further includes step-by-step catheterization instructions arranged and marked on the working tray in accordance with the catheterization procedure.

In some embodiments, steps of the catheterization instructions are marked on the working tray within the preformed sections of the working tray or alongside the preformed sections.

In some embodiments, the storage tray includes an opening in an end of the storage tray. The opening facilitates access to any of the components of the urinary catheter-insertion kit stored in the storage space of the storage tray while working from the working tray.

In some embodiments, the working tray includes a cutout in a corner area of the catheter section formed between a bottom portion and an end portion of the catheter section. The drainage tubing is connected to the urinary catheter through the cutout in the catheter section.

In some embodiments, the cutout allows the drainage tubing to be removed from the storage tray without disconnecting the drainage tubing from the urinary catheter.

In some embodiments, the drainage system further includes a connector. The connector includes a urine-sampling port connecting the urinary catheter to the drainage tubing.

In some embodiments, the urinary catheter-insertion kit further includes a lubricant container including lubricant for the urinary catheter. At least one section of the preformed sections of the working tray includes a lubricant-container section configured to hold the lubricant container.

In some embodiments, the urinary catheter-insertion kit further includes an inflatant container including an inflatant for inflating a balloon of the urinary catheter. At least one section of the preformed sections of the working tray including an inflatant-container section configured to hold the inflatant container.

In some embodiments, the urinary catheter-insertion kit further includes a genital-preparation kit including a package of an antiseptic and one or more swab sticks. At least one section of the preformed sections of the working tray includes a genital-preparation section including a well and one or more angled channels. The well is configured to hold the antiseptic from the package. The one-or-more angled channels are configured to respectively hold the one-or-more swab sticks with their corresponding one or more absorbent heads in the well.

In some embodiments, the well is dimensioned to have a volume commensurate with an entire volume of the antiseptic from the package plus a volume of the one-or-more absorbent heads such that the one-or-more absorbent heads become saturated with the antiseptic when the antiseptic from the package is dispensed in the well.

In some embodiments, the urinary catheter-insertion kit further includes a urine-sampling container disposed in the storage space of the storage tray.

In some embodiments, the urinary catheter-insertion kit further includes protective paperboard disposed in the working tray and a peri-care kit between the protective paperboard and an outer packaging of the urinary catheter-insertion kit. The working tray includes a lip around a perimeter of the working tray configured to hold the protective paperboard. The protective paperboard is configured to protect at least the components of the urinary catheter-insertion kit disposed in the working tray.

Also disclosed herein as an example is a method,which is not part of the invention, for urinary catheterization with a urinary catheter-insertion kit. The method includes an outer packaging-removing step, a leg-opening step, a tray-placing step, a genital area-preparing step, and a catheterizing step. The outer packaging-removing step includes removing an outer packaging from the urinary catheter-insertion kit. The outer packaging-removing step includes baring or exposing a tray assembly including a working tray suspended over a storage tray. The working tray has a number of preformed sections holding a number of components for the urinary catheterization as well as step-by-step catheterization instructions arranged and marked on the working tray in accordance with the urinary catheterization. The leg-opening step includes partially opening legs of a patient to create a space between the legs. The tray-placing step includes placing the tray assembly in the space between the legs of the patient. The tray assembly is contoured for the space between the legs. The genital area-preparing step includes preparing a genital area of the patient using a genital-preparation kit including a package of an antiseptic and one or more swab sticks. The genital area-preparing step includes removing the package of the antiseptic from a genital-preparation section of the working tray closest to the genital area. The genital area-preparing step also includes dispensing the antiseptic into a well of the genital-preparation section in accordance with the catheterization instructions marked on the working tray within or alongside the genital-preparation section. Dispensing the antiseptic into the well saturates one or more absorbent heads of one or more corresponding swab sticks respectively held in one or more angled channels of the genital-preparation section. The catheterizing step includes catheterizing the patient with a urinary catheter, which includes removing the urinary catheter from a catheter section of the working tray farthest from the genital area as well as a drainage system from a covered storage space of the storage tray without disconnecting the urinary catheter from either drainage tubing or a drainage receptacle of the drainage system.

In some embodiments, the tray assembly has a triangular prism shape.

In some embodiments, the tray assembly has a trapezoidal prism shape.

In some embodiments, the tray assembly has a flat-sided teardrop shape. Flat sides of the flat-sided teardrop shape are major sides of the tray assembly.

In some examples, the method,which is not part of the invention, further includes a perianal area-cleaning step. The perianal area-cleaning step includes cleaning a perianal area of the patient with a peri-care kit before the tray-placing step. The perianal area-cleaning step also includes removing the peri-care kit from between the outer packaging of the urinary catheter-insertion kit and a protective paperboard configured to protect the components of the urinary catheter-insertion kit in the working tray under the paperboard.

In some embodiments, the working tray includes a cutout in a corner area of the catheter section formed between a bottom portion and an end portion of the catheter section. The cutout enables removing both the urinary catheter from the catheter section of the working tray and the drainage system from the storage space of the storage tray without disconnecting the urinary catheter from the drainage system.

In some examples, the method,which is not part of the invention, further includes a catheter-lubricating step. The catheter-lubricating step includes removing a lubricant-filled container from a lubricant-container section of the working tray between the genital-preparation section and the catheter section. The catheter-lubricating step also includes dispensing the lubricant from the lubricant-filled container into the lubricant-container section of the working tray in accordance with the catheterization instructions marked on the working tray within or alongside the lubricant-container section. The catheter-lubricating step also includes lubricating the urinary catheter with the lubricant dispensed in the catheter section of the working tray in accordance with the catheterization instructions.

In some examples, the method,which is not part of the invention, further includes a balloon-inflating step. The balloon-inflating step includes removing a saline-filled container from a saline-container section of the working tray between the genital-preparation section and the catheter section. The balloon-inflating step also includes attaching the saline-filled container to the urinary catheter for inflating a balloon of the urinary catheter in accordance with the catheterization instructions marked on the working tray within or alongside the saline-container section.

In some examples, the catheterizing step also includes inserting the urinary catheter into the patient's urethra and completing the balloon-inflating step by inflating the balloon with saline from the saline-filled container in accordance with the catheterization instructions.

These and other features of the concepts provided herein will become more apparent to those of skill in the art in view of the accompanying drawings and following description, which describe particular embodiments of such concepts in greater detail.

### DRAWINGS

FIG. 1 illustrates a urinary catheter-insertion kit including a tray assembly and a number of components for a catheterization procedure in accordance with some embodiments.
FIG. 2 illustrates the tray assembly of FIG. 1 with the components for the catheterization procedure removed therefrom in accordance with some embodiments.
FIG. 3 illustrates a perspective view of the tray assembly in accordance with some embodiments.
FIG. 4 illustrates packing of a number of urinary catheter-insertion kits on a shelf in accordance with some embodiments.
FIG. 5 illustrates the urinary catheter-insertion kit including another tray assembly and the number of components for the catheterization procedure in accordance with some embodiments.
FIG. 6 illustrates the tray assembly of FIG. 5 with the components for the catheterization procedure removed therefrom in accordance with some embodiments.
FIG. 7 illustrates the urinary catheter-insertion kit including yet another tray assembly and the number of components for the catheterization procedure in accordance with some embodiments.
FIG. 8 illustrates the tray assembly of FIG. 7 with the components for the catheterization procedure removed therefrom in accordance with some embodiments.
FIG. 9 illustrates a fastening mechanism for fastening together the tray assembly in accordance with some embodiments.
FIG. 10A illustrates a top or bottom label of the urinary catheter-insertion kit in accordance with some embodiments.
FIG. 10B illustrates a side label of the urinary catheter-insertion kit in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. In addition, any of the foregoing features or steps can, in turn, further include one or more features or steps unless indicated otherwise. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

As set forth above, urinary catheters are used in healthcare facilities such as hospitals to drain urine from patients. However, CAUTIs are the second most common form of HAI. As such, hospitals are interested in ways to cut their CAUTI rates by conforming to strict aseptic techniques as part of their standard of care. But there are many factors that influence a hospital's ability to meet that standard of care. These factors include aspects of healthcare-practitioner training and experience, patient factors (e.g., general health, weight, and anatomy), environmental factors, and characteristics of urinary catheter-insertion kits including components of the kits, various layouts of the components in the kits, and instructions for use of such kits. There is a need in healthcare for an easy, safe, and reliable standard method for inserting a catheter into a patient. Disclosed herein are catheter-insertion kits including urinary catheter insertion kits with integrated instructions for use and methods, which are not part of the invention, thereof.

### Urinary Catheter-Insertion Kits

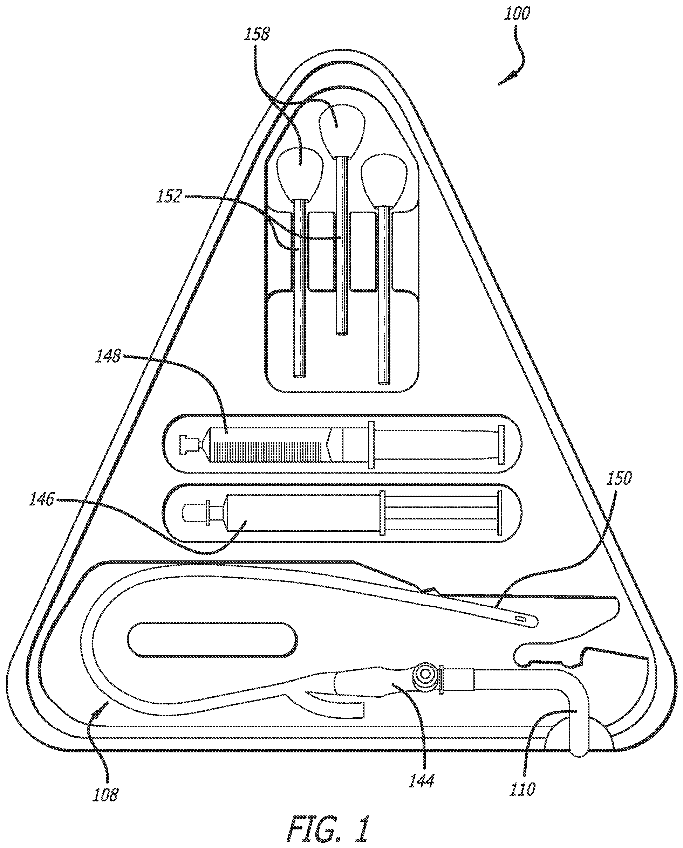

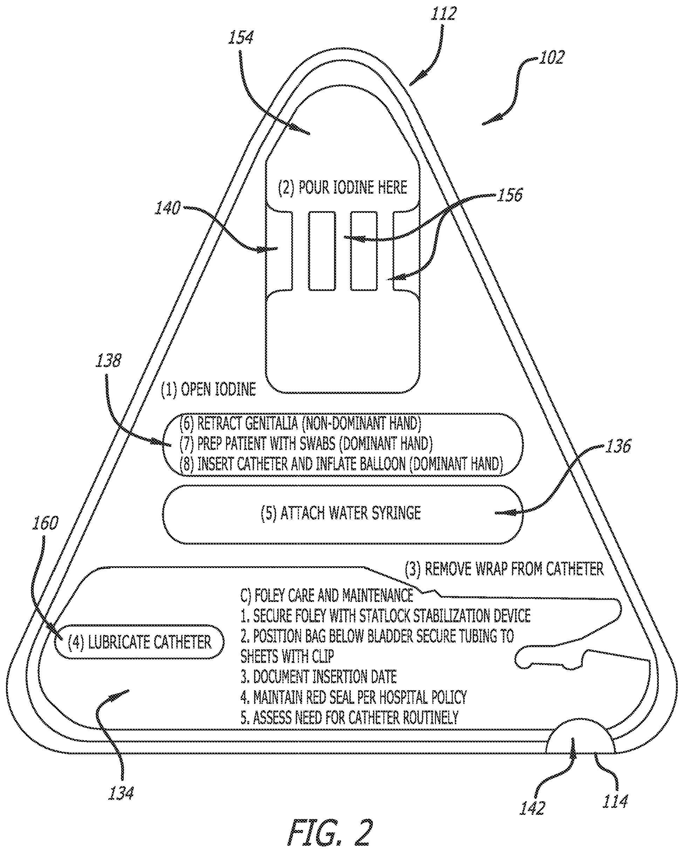

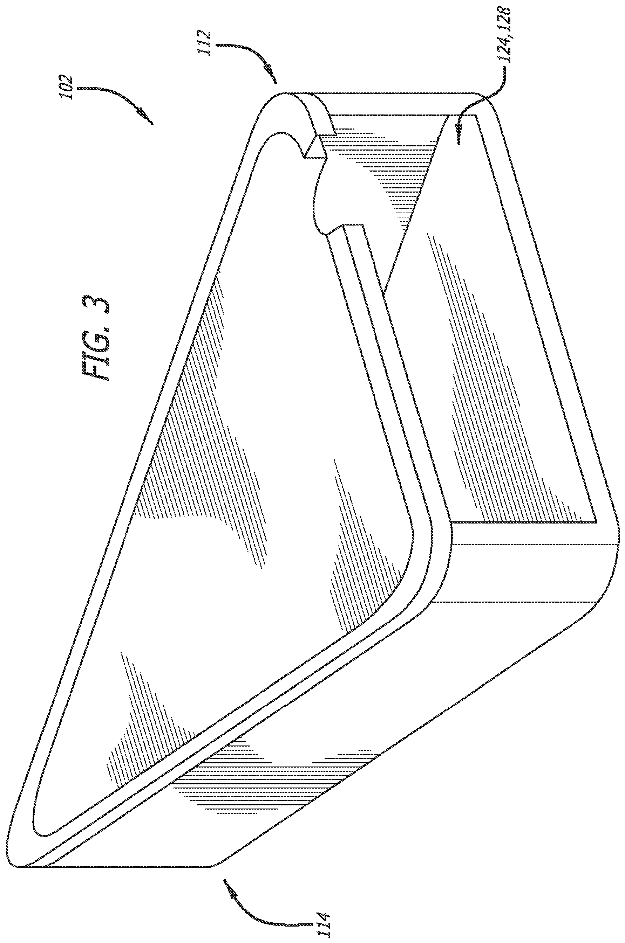

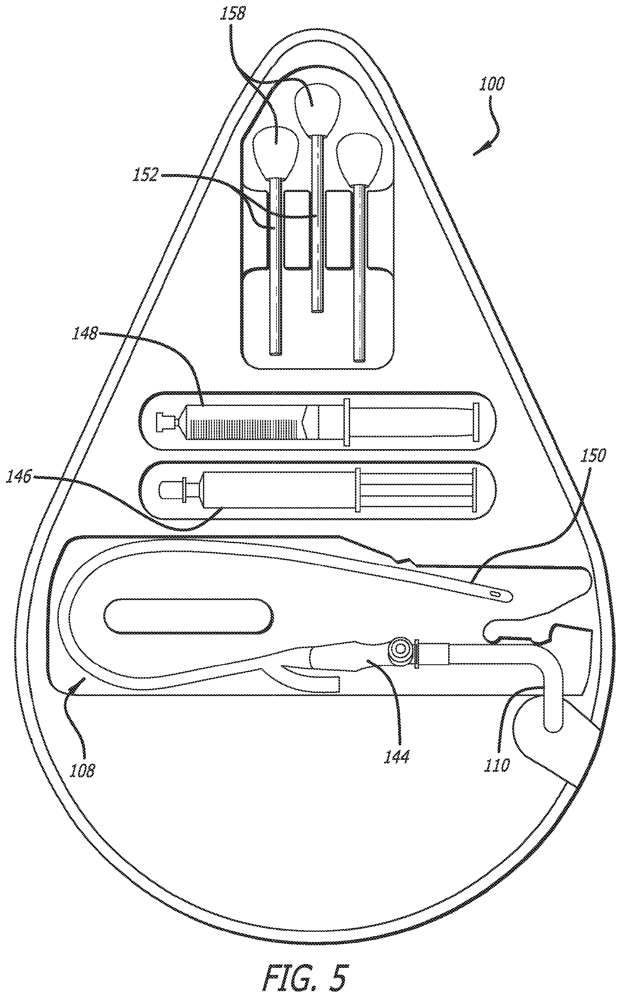

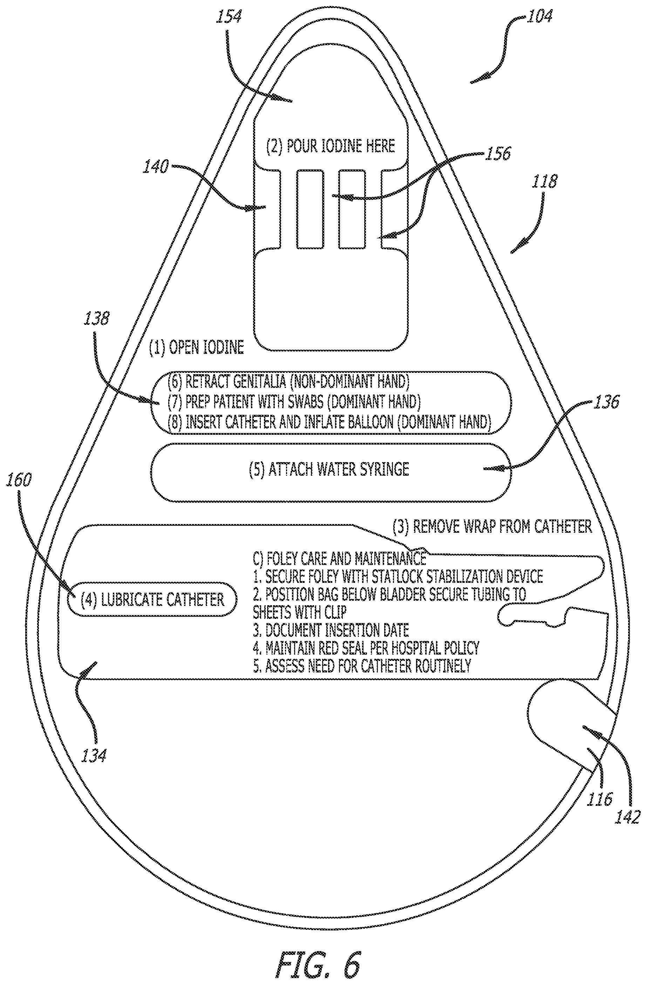

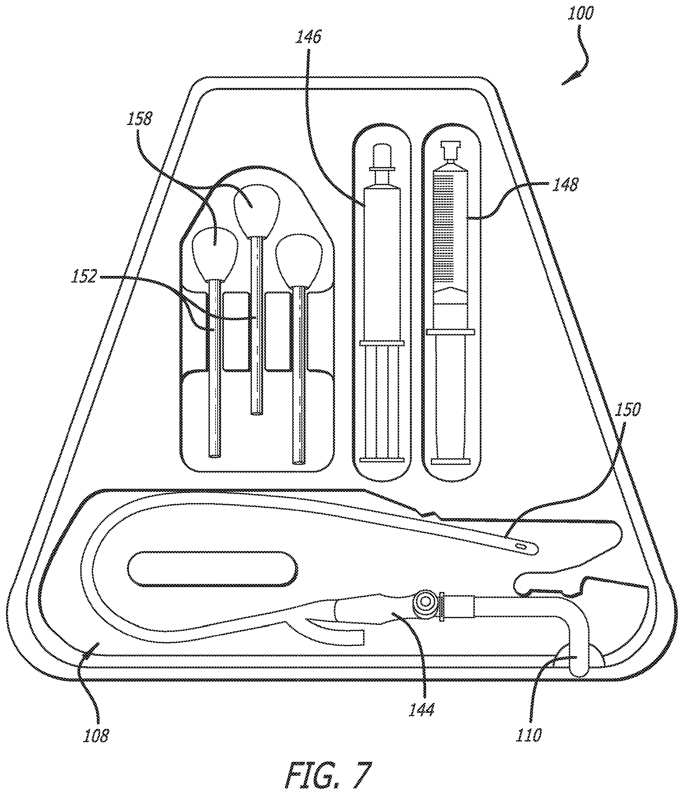

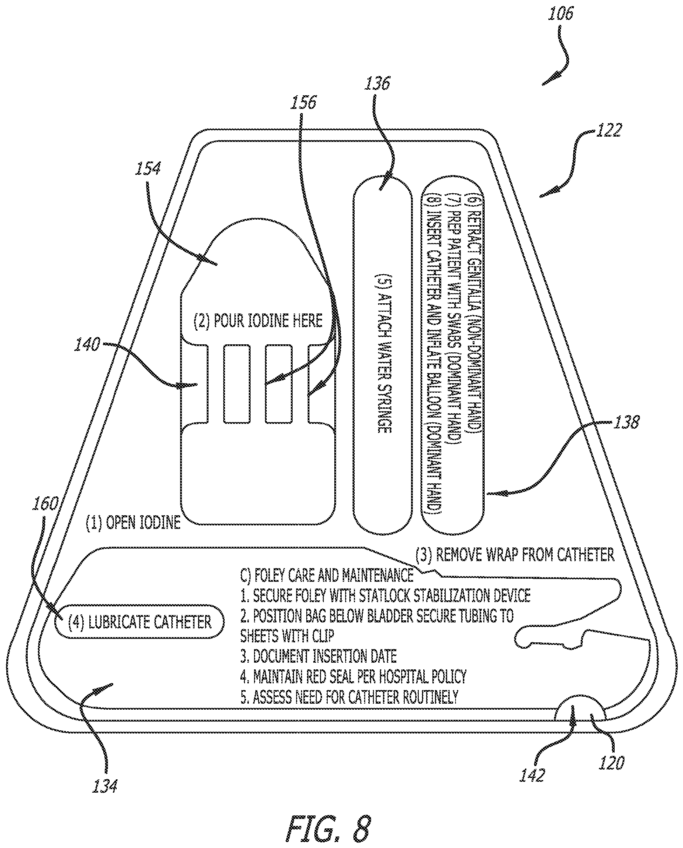

FIGS. 1, 5, and 7 illustrate a urinary catheter-insertion kit 100 including a tray assembly 102, 104, and 106 with a number of components for a catheterization procedure in accordance with some embodiments. FIGS. 2 and 3 illustrate the tray assembly 102 of FIG. 1 with the components for the catheterization procedure removed therefrom in accordance with some embodiments. FIG. 6 illustrate the tray assembly 104 of FIG. 5 with the components for the catheterization procedure removed therefrom in accordance with some embodiments. FIG. 8 illustrate the tray assembly 106 of FIG. 7 with the components for the catheterization procedure removed therefrom in accordance with some embodiments.

For expository expediency, "tray assembly 102" is optionally used herein when generically referring to one or more tray assemblies of the tray assemblies 102, 104, and 106 such as when describing common features among the foregoing tray assemblies. Likewise, "storage tray 112" is optionally used herein when generically referring to one or more storage trays of the storage trays 112, 116, and 120 such as when describing common features among the foregoing storage trays. Further likewise, "working tray 114" is optionally used herein when generically referring to one or more working trays of the working trays 114, 118, and 122 such as when describing common features among the foregoing working trays. Otherwise, specific references are made to the tray assemblies 102, 104, and 106, storage trays 112, 116, and 120, and working trays 114, 118, and 122 such as when describing unique features among the foregoing tray assemblies, working trays, and storage trays. Notably, commonsense contextual interpretation can be used to determine whether a reference to the tray assembly 102, the storage tray 112, or the working tray 114 is a generic or specific reference thereto if differentiation is needed.

The urinary catheter-insertion kit 100 can include the tray assembly 102, a urinary catheter 108 (e.g., a Foley catheter), and a drainage system (not shown) including drainage tubing 110 and a drainage receptacle (e.g., drainage bag, optionally including a urine meter). *(See* U.S. Patent Publication Nos. US 2016/0228676 and US 2017/0216558 for description of the drainage system, which can include a urine meter in addition to the drainage tubing 110 and the drainage receptacle.)

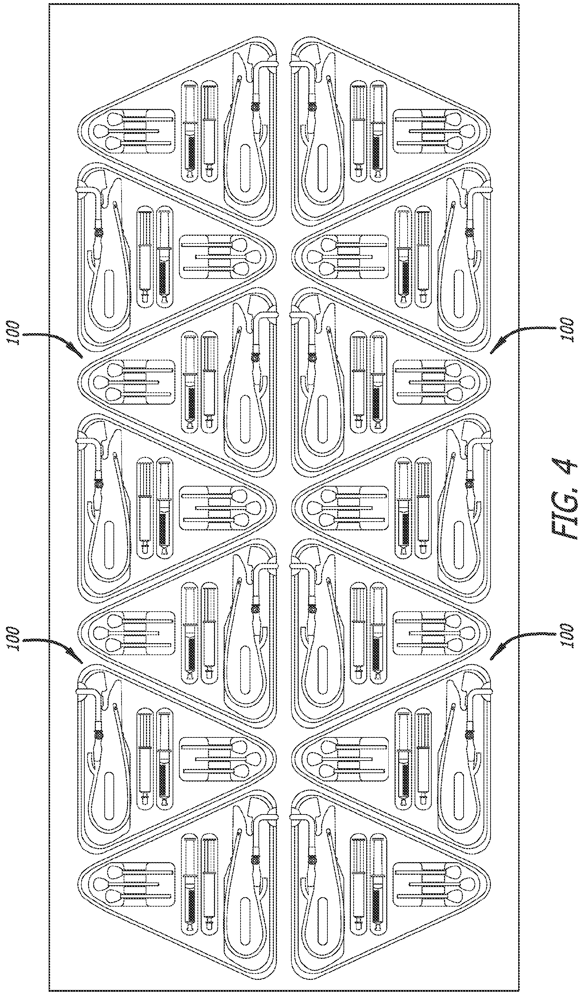

The tray assembly 102 includes a storage tray 112 and a working tray 114. The tray assembly 102 including both the storage tray 112 and the working tray 114 is contoured for a space between partially opened legs of a patient. Indeed, upon at least partially opening legs of a patient in accordance with the leg-opening step of the method ,which is not part of the invention, set forth below, a space between the legs of the patient is created for which the tray assembly 102 is contoured for placement between the legs of the patient. As shown in FIGS. 1-3, the tray assembly 102 can have a triangular prism shape, wherein the working tray 114 nests with the storage tray 112 forming the triangular prism shape of the tray assembly 102. Similarly, the tray assembly 102 can have a flat-sided teardrop shape as exemplified by the tray assembly 104 of FIGS. 5 and 6, wherein flat sides of the flat-sided teardrop shape are major sides of the tray assembly 104. Like that of the foregoing tray assembly 102, a working tray 116 nests with a storage tray 118 to form the flat-sided teardrop shape of the tray assembly 104. Advantageously, a rounded end of the tray assembly 104 provides for additional storage space (i.e., the storage space 128 set forth below) for the drainage system compared to that of the tray assembly 102 or 106. As shown in FIGS. 7 and 8, the tray assembly 102 can have a triangular prism shape as exemplified by the tray assembly 106, wherein a working tray 120 nests with a storage tray 122 forming the triangular prism shape of the tray assembly 106. Such a shape provides additional space between the tray assembly 106 and a genital area of the patient when the tray assembly 106 is disposed in the space between the legs of the patient. If the additional storage space of the tray assembly 104 is desired, the tray assembly 106 can further include the rounded end of the tray assembly 104, thereby providing a truncated flat-sided teardrop shape. Contouring the tray assembly 102 for the space between the partially opened legs of the patient provides patient comfort while also providing convenience to a clinician performing the catheterization procedure. Not only that, but contouring the tray assembly 102 in accordance with the foregoing also allows for efficient packing of the urinary catheter-insertion kits as shown in FIG. 4. Notably, for added comfort, top edges of the working tray 114 can be chamfered or filleted for patient comfort. In addition, corners between minor sides of the storage tray 112 can be rounded for patient comfort.

The storage tray 112 can include an opening 124 in an end of the storage tray 112 as shown in FIG. 3. The opening 124 can be configured to facilitate access to any of the components of the urinary catheter-insertion kit 100 stored or otherwise disposed in the storage tray 112 while working from the working tray 114. For example, until needed, the drainage system can be stored in the storage tray 112 while working from the working tray 114. The opening 124 allows access to a bottom of the working tray 114, if needed, to lift the working tray 114 out of the storage tray 112 while supporting the bottom of the working tray 114. That said, the working tray 114 can be fixed (e.g., fastened, bonded, etc.) to the storage tray 112 in some embodiments. In such embodiments, the urinary catheter-insertion kit 100 is designed such that the working tray 114 remains fixed to the storage tray 112 throughout the catheterization procedure. Indeed, an instruction can be included along with the step-by-step catheterization instructions 160 marked on at least the working tray 114 to not separate the working tray 114 from the storage tray 112.

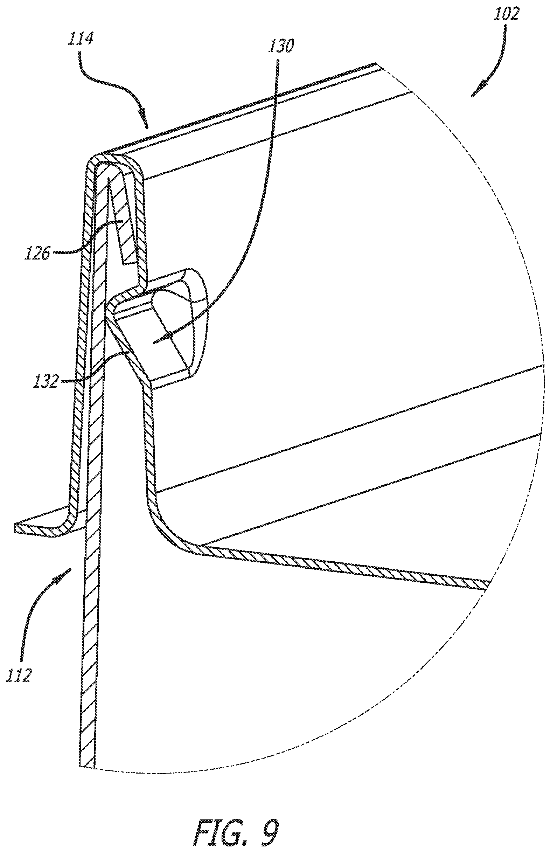

The storage tray 112 can be a folded paperboard storage tray 112. The storage tray 112 can include folded-over longitudinal edges 126 that face inward *(see* FIG. 9) or outward from the storage tray 112 such that the folded-over longitudinal edges 126 can engage the protrusions 132 of the working tray 114 set forth below. Such folded-over longitudinal edges thereby form a storage-tray portion of a fastening mechanism for fastening together the working tray 114 and the storage tray 112. As an alternative to the folded-over longitudinal edges 126, the storage tray 112 can include a number of folded-over tabs that face inward or outward from the storage tray 112 such the tabs can engage the protrusions 132 of the working tray 114, thereby forming an alternative storage-tray portion of the fastening mechanism for fastening together the working tray 114 and the storage tray 112.

The working tray 114 can be configured to nest with the storage tray 112 by suspending the working tray 114 from the storage tray 112 as shown in FIGS. 3 and 9. Indeed, the working tray 114 is suspended over the storage tray 112 in the tray assembly 102, thereby forming a covered storage space 128 in the storage tray 112. The working tray 114 can be molded plastic and include a number of indentations 130 molded into either side of a pair of longitudinal sides of an overhanging edge of the working tray 114. The number of indentations 130 correspondingly provide a number of protrusions 132 between the longitudinal sides of the overhanging edge that can face outward (*see* FIG. 9) or inward from the working tray 114. Such protrusions form a working-tray portion of the fastening mechanism for fastening together the working tray 114 and the storage tray 112. Indeed, as shown in FIG. 9, the working tray 114 can be configured to accept insertion of the folded-over longitudinal edges 126 or tabs of the storage tray 112 between the longitudinal sides of the overhanging edge where the protrusions 132 engage the folded-over longitudinal edges 126 or tabs of the storage tray 112, thereby fastening together the working tray 114 and the storage tray 112 and preventing separation thereof. Notably, the overhanging edge can be dimensioned to provide the folded-over longitudinal edges 126 or tabs of the storage tray 112 room to expand under a spring force present within folds of the folded-over longitudinal edges 126 or tabs created by forcing the folded-over longitudinal edges 126 or tabs between the longitudinal sides of the overhanging edge of the working tray 114. In addition, the protrusions 132 can protrude as sharply as molding (e.g., thermoforming) allows making engaging surfaces of the protrusions 132 as flat as possible for engaging the folded-over longitudinal edges 126 or tabs of the storage tray 112. The foregoing fastening mechanism obviates a need for a secondary mechanism (e.g., bonding) for fastening the working tray 114 and the storage tray 112 together.

At least the working tray 114 can include a number of preformed sections configured to respectively hold a number of components of the urinary catheter-insertion kit 100. Indeed, in a ready-to-use state of the urinary catheter-insertion kit 100, the preformed sections hold the components of the urinary catheter-insertion kit 100 for which the preformed sections are configured. The preformed sections of the working tray 114 can include, but are not limited to, a catheter section 134 configured to hold the urinary catheter 108, a lubricant-container section 136 configured to hold the lubricant container 146 set forth below, an inflatant-container section 138 configured to hold the inflatant container 148 set forth below, a genital-preparation section 140 configured to hold the genital-preparation kit set forth below. Advantageously, the preformed sections of the working tray 114 can be arranged in accordance with the catheterization procedure. That is, earlier steps in the catheterization procedure utilize the components in the preformed sections of the working tray 114 closer to a genital area of the patient; later steps in the catheterization procedure utilize the components in the preformed sections of the working tray 114 farther from the genital area. Coupled with the step-by-step catheterization instructions 160 set forth below, which instructions are arranged with the preformed sections of the working tray 114 in accordance with the catheterization procedure, the urinary catheter-insertion kit 100 provides an easy or stress-free, safe, and reliable standard method,which is not part of the invention, for inserting the urinary catheter 108 into the patient.

The catheter section 134 is configured to hold the urinary catheter 108. Indeed, in a ready-to-use state of the urinary catheter-insertion kit 100, the catheter section 134 holds the urinary catheter 108 for which the catheter section 134 is configured. A cutout 142 in a corner area of the catheter section 134 formed between a bottom portion and an end portion of the catheter section 134 can be configured to allow, for example, the drainage tubing 110 of the drainage system to pass from the storage tray 112 to the working tray 114 by way of a bottom of the cutout 142 when the urinary catheter 108 is pre-connected to the drainage system, optionally, through a connector 144 including a urine-sampling port, and the drainage tubing 110 is stored in the storage tray 112. A side of the cutout 142 in the corner area of the catheter section 134 can be configured to allow the drainage tubing 110 to be removed from the storage tray 112 without disconnecting the drainage tubing 110 from the urinary catheter 108 when the drainage system and the urinary catheter 108 are pre-connected in the urinary catheter-insertion kit 100. In contrast, if the end portion of the catheter section 134 is intact and the cutout 142 consists of only the bottom portion of the catheter section 134 (i.e., the cutout 142 is merely a hole in the bottom portion of the catheter section 134 ), the drainage tubing 110 and, subsequently, the urinary catheter 108 would have to be pulled through the cutout 142 when the drainage system and the urinary catheter 108 are pre-connected, which risks contamination (e.g., touch contamination) of the urinary catheter 108. Alternatively, the drainage tubing 110 would have to be disconnected from the urinary catheter 108 when the drainage system and the urinary catheter 108 are pre-connected, which also risks contamination of the urinary catheter 108 by way of either disconnecting the drainage tubing 110 from the urinary catheter 108 or reconnecting the drainage tubing 110 to the urinary catheter 108. This is contrary to the purpose of pre-connecting the urinary catheter 108 and the drainage system, which is to minimize any potential contamination.

The lubricant-container section 136 is configured to hold a lubricant container 146 (e.g., syringe, packet, pod, vial, etc.) including, containing, or filled with lubricant for the urinary catheter 108. Indeed, in a ready-to-use state of the urinary catheter-insertion kit 100, the lubricant-container section 136 holds the lubricant container 146 for which the lubricant-container section 136 is configured. The lubricant-container section 136 can be preformed to accommodate the lubricant container 146 (e.g., a lubricant-syringe section, a lubricant-packet section, a lubricant-pod section, a lubricant-vial section, etc.); however, the lubricant-container section 136 need not conform to every feature of the lubricant container 146. For example, if the lubricant container 146 is a syringe, the lubricant-container section 136 (e.g., the lubricant-syringe section) need not include a recess for a flange of the syringe. That is, the lubricant-container section 136 can have a constant width or depth commensurate with at least the flange of the syringe to accommodate the syringe. Indeed, the lubricant-container section 136 can have a constant width or depth commensurate with the greatest width or diameter of any type of the lubricant container 146 set forth herein. Advantageously, the lubricant-container section 136 can include a lubricant well or double as a lubricant well configured to hold lubricant dispensed therein for lubricating the urinary catheter 108. Alternatively, the working tray 114 includes another section set forth herein (e.g., the catheter section 134) including the lubricant well or doubling as the lubricant well. Further alternatively, the working tray 114 includes a dedicated lubricant-well section configured to hold the lubricant dispensed therein for lubricating the urinary catheter 108.

The inflatant-container section 138 is configured to hold an inflatant container 148 (e.g., syringe) including, containing, or filled with an inflatant (e.g., saline) for inflating a balloon 150 of the urinary catheter 108. Indeed, in a ready-to-use state of the urinary catheter-insertion kit 100, the inflatant-container section 138 holds the inflatant container 148 for which the inflatant-container section 138 is configured. The inflatant-container section 138 can be preformed to accommodate the inflatant container 148; however, the inflatant-container section 138 need not conform to every feature of the inflatant container 148. For example, if the inflatant container 148 is a syringe, the inflatant-container section 138 (e.g., an inflatant-syringe section) need not include a recess for a flange of the syringe. That is, the inflatant-container section 138 can have a constant width or depth commensurate with at least the flange of the syringe to accommodate the syringe. Indeed, the inflatant-container section 138 can have a constant width or depth commensurate with the greatest width or diameter of any type of the inflatant container 148 set forth herein.

As shown, the lubricant-container section 136 and the inflatant-container section 138 can be commensurate in shape and size. For example, the lubricant-container section 136 and the inflatant-container section 138 can have a same length, width, depth from common top surface of the working tray 114, or a combination thereof. Alternatively, each of the lubricant-container section 136 and the inflatant-container section 138 can have a different shape or size as shown in FIGS. A, 2, and 5-8.

The genital-preparation section 140 is configured to hold a genital-preparation kit including a package of an antiseptic (not shown) and one or more swab sticks 152. (*See* U.S. Patent Publication Nos. US 2016/0228676 and US 2017/0216558 for description of the genital-preparation kit.) Indeed, in a ready-to-use state of the urinary catheter-insertion kit 100, the genital-preparation section 140 holds the genital-preparation kit for which the genital-preparation section 140 is configured. Advantageously, the genital-preparation section 140 can include an antiseptic well 154 or double as an antiseptic well to hold the antiseptic (e.g., povidone-iodine solution) from the package thereof. The genital-preparation section 140 can also include one or more angled channels 156 with snap-in tabs configured to respectively hold the one-or-more swab sticks 152 with their corresponding one or more absorbent heads 158 in the antiseptic well 154. The antiseptic well 154 is dimensioned to have a volume commensurate with an entire volume of the antiseptic from the package plus a volume of the one-or-more absorbent heads 158 such that the one-or-more absorbent heads 158 become saturated with the antiseptic when the antiseptic from the package is dispensed in the antiseptic well 154.

While not shown, the preformed sections of the working tray 114 can further include a urine sampling-container section configured to hold a urine-sampling container. Alternatively, the storage tray 112 can be configured to hold the urine-sampling container instead of the working tray 114. Indeed, in a ready-to-use state of the urinary catheter-insertion kit 100, the urine sampling-container section holds the urine-sampling container for which the urine sampling-container section is configured. Configuring the storage tray 112 to hold the urine-sampling container instead of the working tray 114 can minimize a height of the working tray 114.

The urinary catheter-insertion kit 100 can further include step-by-step catheterization instructions 160 arranged and marked on (e.g., printed on, imprinted in, etc. ) at least the working tray 114 for the catheterization procedure with the urinary catheter-insertion kit 100. At least some of the step-by-step catheterization instructions 160 can be placed in a location (e.g., within one or more of the preformed sections of the working tray 114, alongside one or more of the preformed sections of the working tray 114, etc.) that expressly or implicitly suggests by way of the location how to perform a step of the catheterization procedure. For example, the genital-preparation section 140 can include an express instruction within the genital-preparation section 140 such as in the antiseptic well 154 for dispensing the antiseptic from the package thereof into the antiseptic well 154. For example, the catheter section 134 can include an implicit instruction within the catheter section 134 for dispensing the lubricant into the catheter section 134 for lubricating the urinary catheter 108. At least some of the step-by-step catheterization instructions 160 can be revealed in step with steps of the catheterization procedure. For example, removing the package of the antiseptic from the genital-preparation section 140 can reveal the instruction to dispense the antiseptic from the package into the antiseptic well 154 of the genital-preparation section 140 for saturating the one-or-more absorbent heads 158 of the one-or-more swab sticks 152 with the antiseptic. For example, removing the lubricant container 146 from the lubricant-container section 136 can reveal the instruction to attach the inflatant container 148 for inflating the balloon 150 of the urinary catheter 108. Revealing steps of the step-by-step catheterization instructions 160 in this way does not overwhelm the clinician preforming the catheterization with too many steps at once.

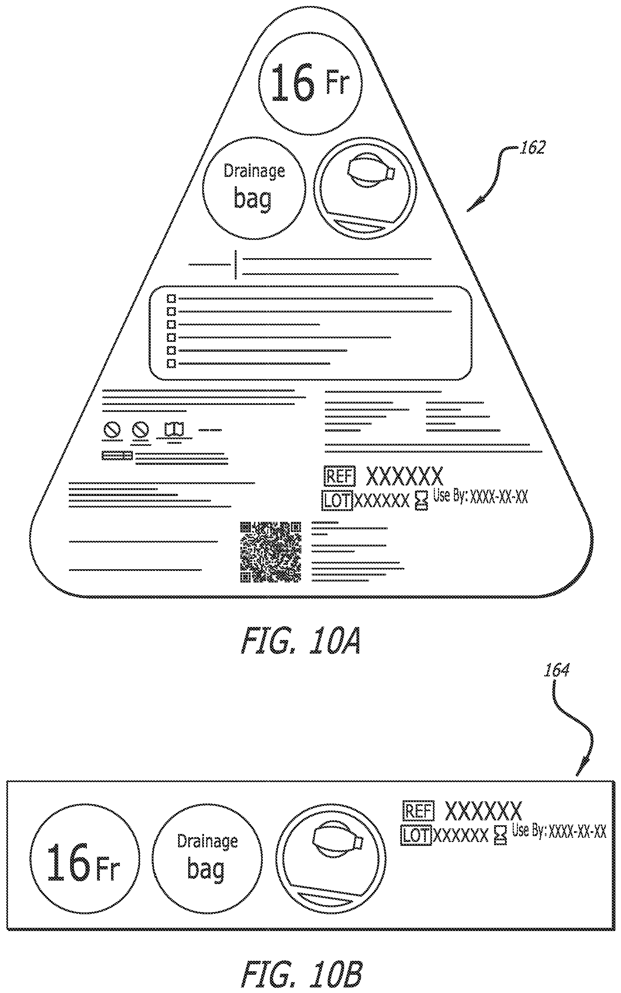

FIGS. 10A and 10B illustrate labels of the urinary catheter-insertion kit in accordance with some embodiments.

As shown, the urinary catheter-insertion kit 100 can include a top or bottom label 162 and a side label 164 for each side of the tray assembly 102. The labels 162 and 164 can include information on the urinary catheter-insertion kit 100 such as information about the components of the urinary catheter-insertion kit 100. For example, the top or bottom label can indicate a size of urinary catheter 108.

The urinary catheter-insertion kit 100 can further include protective paperboard (not shown) configured to protect at least the components of the working tray 114. The working tray 114 can include a lip around a perimeter of the working tray 114 configured to hold the paperboard.

The urinary catheter-insertion kit 100 can further include a peri-care kit (not shown). (*See* U.S. Patent Publication Nos. US 2016/0228676 and US 2017/0216558 for description of the peri-care kit.) The urinary catheter-insertion kit 100 can be configured to include the peri-care kit between the paperboard and an outer packaging (e.g., Tyvek^{®}) of the urinary catheter-insertion kit 100.

Additional components not shown in FIGS. 1, 5, and 7 or described above that can be useful in the urinary catheter-insertion kit 100 include, but are not limited to, any one or more components selected from a stabilization device (e.g., C. R. Bard, Inc.'s StatLock^{®} Foley stabilization device), prepping balls (e.g., absorbent cotton balls), forceps, a label that can be filled out with catheterization or urine-sampling details, a fenestrated drape to place over the patient, an underpad to place under the patient, gloves (e.g., a package of latex or latex-free gloves), a sterile wrap (e.g., Central Supply Room ["CSR"] wrap) to wrap around the working and storage trays 112 and 114, a belly band (e.g., to hold the sterile wrap in place around the working and storage trays 112 and 114), hand sanitizer, moist towelettes (e.g., a package of castile-soap towelettes), additional instructions to those marked on at least the working tray 114 (e.g., an instruction sheet for a clinician, an instruction pamphlet for the patient, etc.), a checklist of safety considerations, a patient information chart, an insert sheet, and a packaging label. Such additional components can be found in U.S. Patent Publication Nos. US 2016/0228676 and US 2017/0216558.

### Methods (which are not part of the invention)

Methods of urinary catheterization with the urinary catheter-insertion kit 100 can include one or more steps selected from an outer packaging-removing step, a leg-opening step, a perianal area-cleaning step, a tray-placing step, a genital area-preparing step, a catheter-lubricating step, a catheterizing step, and a balloon-inflating step.

The outer packaging-removing step can include removing the outer packaging from the urinary catheter-insertion kit 100. The outer packaging-removing step can include baring or exposing the tray assembly 102, which, as set forth above, includes the working tray 114 suspended over the storage tray 112. Again, the working tray 114 has a number of preformed sections that hold the components for the urinary catheterization as well as the step-by-step catheterization instructions 160 arranged and marked on the working tray 114 in accordance with the urinary catheterization. The outer packaging-removing step can also include baring or exposing the protective paperboard configured to protect the components of the urinary catheter-insertion kit 100 in the working tray 114 under the paperboard.

The leg-opening step can include partially opening legs of a patient to create a space between the legs.

The perianal area-cleaning step can include cleaning a perianal area of the patient with the peri-care kit before the tray-placing step. The perianal area-cleaning step can also include removing the peri-care kit from between the outer packaging of the urinary catheter-insertion kit 100 and the protective paperboard configured to protect the components of the urinary catheter-insertion kit 100 in the working tray 114 under the paperboard.

The tray-placing step can includes placing the tray assembly 102 in the space between the legs of the patient. Again, the tray assembly 102 is contoured for the space between the legs. Indeed, as set forth above, the tray assembly 102 can have the triangular prism shape of the tray assembly 102 of FIGS. 1-3, the trapezoidal prism shape of the tray assembly 104 of FIGS. 5 and 6, or the flat-sided teardrop shape of the tray assembly 106 of FIGS. 7 and 8.

The genital area-preparing step can include preparing a genital area of the patient using the genital-preparation kit, which includes the package of the antiseptic and the one-or-more swab sticks 152. The genital area-preparing step can also include removing the package of the antiseptic from the genital-preparation section 140 of the working tray 114 closest to the genital area. The genital area-preparing step can also include dispensing the antiseptic into the antiseptic well 154 of the genital-preparation section 140 in accordance with the step-by-step catheterization instructions 160 marked on the working tray 114 within or alongside the genital-preparation section 140. These instructions are shown in FIGS. 2, 6, and 8 as the first and second steps of the step-by-step catheterization instructions 160 marked on the working tray 114 about the genital-preparation section 140. Dispensing the antiseptic into the antiseptic well 154 saturates the one-or-more absorbent heads 158 of the one-or-more swab sticks 152 respectively held in the one-or-more angled channels 156 of the genital-preparation section 140.

The genital area-preparing step can also include retracting genitalia of the patient using a non-dominant hand and swabbing the genitalia with the antiseptic using a dominant hand in accordance with the step-by-step catheterization instructions 160 imprinted directly on the working tray 114. These instructions are shown in FIGS. 2, 6, and 8 as the sixth, seventh, and eighth steps of the step-by-step catheterization instructions 160 marked on the working tray 114 about the lubricant-container section 136.

The catheter-lubricating step can include removing the lubricant-filled container from the lubricant-container section 136 of the working tray 114 between the genital-preparation section 140 and the catheter section 134. The catheter-lubricating step can also include dispensing the lubricant from the lubricant-filled container into the lubricant-container section 136 of the working tray 114 in accordance with the step-by-step catheterization instructions 160 marked on the working tray 114 within or alongside the lubricant-container section 136. These instructions are shown in a summarized form in FIGS. 2, 6, and 8 as the fourth step of the step-by-step catheterization instructions 160 marked on the working tray 114 about the catheter section 134. The catheter-lubricating step can also include lubricating the urinary catheter 108 with the lubricant dispensed in the catheter section 134 of the working tray 114 in accordance with the step-by-step catheterization instructions 160.

The catheterizing step can include catheterizing the patient with the urinary catheter 108, which can include removing the urinary catheter 108 from the catheter section 134 of the working tray 114 farthest from the genital area as well as the drainage system from the storage space 128 of the storage tray 112 without disconnecting the urinary catheter 108 from either the drainage tubing 110 or the drainage receptacle of the drainage system. As set forth above, the working tray 114 includes the cutout 142 in the corner area of the catheter section 134 formed between the bottom portion and the end portion of the catheter section 134. The cutout 142 enables removing both the urinary catheter 108 from the catheter section 134 of the working tray 114 and the drainage system from the storage space 128 of the storage tray 112 without disconnecting the urinary catheter 108 from the drainage system.

The catheterizing step can also include inserting the urinary catheter 108 into the patient's urethra and inflating the balloon 150 with inflatant (e.g., saline) from an inflatant-filled container (e.g., saline-filled container) in accordance with the step-by-step catheterization instructions 160. A balloon-inflating step such as the foregoing can include removing the inflatant-filled container from the inflatant-container section 138 of the working tray 114 between the genital-preparation section 140 and the catheter section 134. The balloon-inflating step can also include attaching the inflatant-filled container to the urinary catheter 108 for inflating the balloon 150 of the urinary catheter 108 in accordance with the step-by-step catheterization instructions 160 marked on the working tray 114 within or alongside the saline-container section.

While some particular embodiments have been disclosed herein, and while the particular embodiments have been disclosed in some detail, it is not the intention for the particular embodiments to limit the scope of the concepts provided herein. Additional adaptations or modifications can appear to those of ordinary skill in the art, and, in broader aspects, these adaptations or modifications are encompassed as well. Accordingly, departures may be made from the particular embodiments disclosed herein without departing from the scope of the concepts provided herein.

## Claims

1. A urinary catheter-insertion kit (100) for a catheterization procedure, comprising:
a tray assembly (102, 104, 106) contoured for a space between partially opened legs of a patient, the tray assembly (102, 104, 106) including:
a storage tray (112, 116, 120); and
a working tray (114, 118, 122) including a plurality of preformed sections holding a plurality of components of the urinary catheter-insertion kit (100), the working tray (114, 118, 122) suspended over the storage tray (112, 116, 120) in the tray assembly (102, 104, 106), thereby forming a covered storage space (128) in the storage tray (112, 116, 120);
a urinary catheter (108) disposed in a catheter section (134) of the preformed sections; and
a drainage system disposed in the storage space (128), the drainage system including a drainage receptacle and drainage tubing (110) connected to the urinary catheter (108),
**characterised in that**,
the preformed sections of the working tray (114, 118, 122) are arranged in accordance with the catheterization procedure, earlier steps in the catheterization procedure utilizing the components in the preformed sections closer to a genital area of the patient and later steps in the catheterization procedure utilizing the components in the preformed sections farther from the genital area.

2. The urinary catheter-insertion kit (100) according to claim 1, wherein the tray assembly (102, 104, 106) has:
a triangular prism shape,
a trapezoidal prism shape, or
a flat-sided teardrop shape, flat sides of the flat-sided teardrop shape being major sides of the tray assembly (102, 104, 106).

3. The urinary catheter-insertion kit (100) according to claim 1 or 2, wherein top edges of the working tray (114, 118, 122) are chamfered or filleted for patient comfort, and/or
wherein corners between minor sides of the storage try are rounded for patient comfort.

4. The urinary catheter-insertion kit (100) according to any claim of claims 1-3, wherein the working tray (114, 118, 122) is molded plastic, and/or
wherein the storage tray (112, 116, 120) is folded paperboard.

5. The urinary catheter-insertion kit (100) according to any claim of claims 1-4, further comprising step-by-step catheterization instructions arranged and marked on the working tray (114, 118, 122) in accordance with the catheterization procedure, preferably
wherein steps of the catheterization instructions are marked on the working tray (114, 118, 122) within the preformed sections of the working tray (114, 118, 122) or alongside the preformed sections.

6. The urinary catheter-insertion kit (100) according to any claim of claims 1-5, wherein the storage tray (112, 116, 120) includes an opening (124) in an end of the storage tray (112, 116, 120) to facilitate access to any of the components of the urinary catheter-insertion kit (100) stored in the storage space of the storage tray (112, 116, 120) while working from the working tray (114, 118, 122).

7. The urinary catheter-insertion kit (100) according to any claim of claims 1-6, wherein the working tray (114, 118, 122) includes a cutout (142) in a corner area of the catheter section (134) formed between a bottom portion and an end portion of the catheter section (134), the drainage tubing (110) connected to the urinary catheter (108) through the cutout (142) in the catheter section (134).

8. The urinary catheter-insertion kit (100) according to claim 7, wherein the cutout (142) allows the drainage tubing (110) to be removed from the storage tray (112, 116, 120) without disconnecting the drainage tubing (110) from the urinary catheter (108).

9. The urinary catheter-insertion kit (100) according to any claim of claims 1-8, the drainage system further comprising a connector (144) including a urine-sampling port connecting the urinary catheter (108) to the drainage tubing (110).

10. The urinary catheter-insertion kit (100) according to any claim of claims 1-9, further comprising a lubricant container (146) including lubricant for the urinary catheter (108), at least one section of the preformed sections of the working tray (114, 118, 122) including a lubricant-container section (138) configured to hold the lubricant container (146).

11. The urinary catheter-insertion kit (100) according to any claim of claims 1-10, further comprising an inflatant container (148) including an inflatant for inflating a balloon of the urinary catheter (108), at least one section of the preformed sections of the working tray (114, 118, 122) including an inflatant-container section (138) configured to hold the inflatant container (148).

12. The urinary catheter-insertion kit (100) according to any claim of claims 1-11, further comprising a genital-preparation kit including a package of an antiseptic and one or more swab sticks (152), at least one section of the preformed sections of the working tray (114, 118, 122) including a genital-preparation section (140) configured with a well (154) to hold the antiseptic from the package and one or more angled channels (156) to respectively hold the one-or-more swab sticks (152) with their corresponding one or more absorbent heads (158) in the well.

13. The urinary catheter-insertion kit (100) according to claim 12, wherein the well (154) is dimensioned to have a volume commensurate with an entire volume of the antiseptic from the package plus a volume of the one-or-more absorbent heads (158) such that the one-or-more absorbent heads (158) become saturated with the antiseptic when the antiseptic from the package is dispensed in the well (154).

14. The urinary catheter-insertion kit (100) according to any claim of claims 1-13, further comprising a urine-sampling container disposed in the storage space of the storage tray (112, 116, 120).

15. The urinary catheter-insertion kit (100) according to any claim of claims 1-14, further comprising:
protective paperboard configured to protect at least the components of the urinary catheter-insertion kit (100) disposed in the working tray (114, 118, 122), the working tray (114, 118, 122) including a lip around a perimeter of the working tray (114, 118, 122) configured to hold the protective paperboard; and
a peri-care kit between the protective paperboard and an outer packaging of the urinary catheter-insertion kit (100).

## Patentansprüche

1. Harnblasenkathetereinführungskit (100) für eine Katheterisierungsprozedur, umfassend:
eine Tablettanordnung (102, 104, 106), die für einen Raum zwischen teilweise geöffneten Beinen eines Patienten konturiert ist, wobei die Tablettanordnung (102, 104, 106) Folgendes einschließt: ein Aufbewahrungstablett (112, 116, 120); und
ein Arbeitstablett (114, 118, 122), das eine Vielzahl von vorgeformten Abschnitten einschließt, die eine Vielzahl von Komponenten des Harnblasenkathetereinführungskits (100) halten, wobei das Arbeitstablett (114, 118, 122) in der Tablettanordnung (102, 104, 106) über dem Aufbewahrungstablett (112, 116, 120) aufgehängt ist, wodurch ein abgedeckter Aufbewahrungsraum (128) in dem Aufbewahrungstablett (112, 116, 120) gebildet wird;
einen Harnblasenkatheter (108), der in einem Katheterabschnitt (134) der vorgeformten Abschnitte angeordnet ist; und
ein Entwässerungssystem, das in dem Aufbewahrungsraum (128) angeordnet ist, wobei das Entwässerungssystem einen Entwässerungsbehälter und einen Entwässerungsschlauch (110) einschließt, der mit dem Harnblasenkatheter (108) geschaltet ist,
**dadurch gekennzeichnet, dass**
die vorgeformten Abschnitte des Arbeitstabletts (114, 118, 122) in Übereinstimmung mit der Katheterisierungsprozedur angeordnet sind, wobei frühere Schritte in der Katheterisierungsprozedur die Komponenten in den vorgeformten Abschnitten näher an einem Genitalbereich des Patienten nutzen und spätere Schritte in der Katheterisierungsprozedur die Komponenten in den vorgeformten Abschnitten weiter entfernt von dem Genitalbereich nutzen.

2. Harnblasenkathetereinführungskit (100) nach Anspruch 1, wobei die Tablettanordnung (102, 104, 106) Folgendes aufweist:
eine dreieckige Prismenform,
eine trapezförmige Prismenform, oder
eine flachseitige Tropfenform, wobei flache Seiten der flachseitigen Tropfenform Hauptseiten der Tablettanordnung (102, 104, 106) sind.

3. Harnblasenkathetereinführungskit (100) nach Anspruch 1 oder 2, wobei obere Kanten des Arbeitstabletts (114, 118, 122) für Patientenkomfort abgeschrägt oder verrundet sind, und/oder
wobei Ecken zwischen Nebenseiten des Aufbewahrungstabletts für Patientenkomfort abgerundet sind.

4. Harnblasenkathetereinführungskit (100) nach einem Anspruch der Ansprüche 1-3, wobei das Arbeitstablett (114, 118, 122) geformter Kunststoff ist, und/oder
wobei das Aufbewahrungstablett (112, 116, 120) gefalteter Karton ist.

5. Harnblasenkathetereinführungskit (100) nach einem Anspruch der Ansprüche 1-4, das weiter Schritt-für-Schritt-Katheterisierungsanweisungen umfasst, die auf dem Arbeitstablett (114, 118, 122) in Übereinstimmung mit der Katheterisierungsprozedur angeordnet und markiert sind, bevorzugt
wobei Schritte der Katheterisierungsanweisungen auf dem Arbeitstablett (114, 118, 122) innerhalb der vorgeformten Abschnitte des Arbeitstabletts (114, 118, 122) oder neben den vorgeformten Abschnitten markiert sind.

6. Harnblasenkathetereinführungskit (100) nach einem Anspruch der Ansprüche 1-5, wobei das Aufbewahrungstablett (112, 116, 120) eine Öffnung (124) an einem Ende des Aufbewahrungstabletts (112, 116, 120) einschließt, um den Zugang zu einer beliebigen der Komponenten des Harnblasenkathetereinführungskits (100) zu erleichtern, die in dem Aufbewahrungsraum des Aufbewahrungstabletts (112, 116, 120) gelagert sind, während von dem Arbeitstablett (114, 118, 122) aus gearbeitet wird.

7. Harnblasenkathetereinführungskit (100) nach einem Anspruch der Ansprüche 1-6, wobei das Arbeitstablett (114, 118, 122) eine Aussparung (142) in einem Eckbereich des Katheterabschnitts (134) einschließt, der zwischen einem Bodenabschnitt und einem Endabschnitt des Katheterabschnitts (134) gebildet ist, wobei der Entwässerungsschlauch (110) durch die Aussparung (142) in dem Katheterabschnitt (134) mit dem Harnblasenkatheter (108) geschaltet ist.

8. Harnblasenkathetereinführungskit (100) nach Anspruch 7, wobei die Aussparung (142) es ermöglicht, den Entwässerungsschlauch (110) aus dem Aufbewahrungstablett (112, 116, 120) zu entfernen, ohne den Entwässerungsschlauch (110) von dem Harnblasenkatheter (108) zu trennen.

9. Harnblasenkathetereinführungskit (100) nach einem Anspruch der Ansprüche 1-8, wobei das Entwässerungssystem weiter einen Konnektor (144) umfasst, der einen Urinprobenentnahmeport einschließt, der den Harnblasenkatheter (108) mit dem Entwässerungsschlauch (110) schaltet.

10. Harnblasenkathetereinführungskit (100) nach einem Anspruch der Ansprüche 1-9, das weiter einen Gleitmittelbehälter (146) umfasst, der Gleitmittel für den Harnblasenkatheter (108) einschließt, wobei zumindest ein Abschnitt der vorgeformten Abschnitte des Arbeitstabletts (114, 118, 122) einen Gleitmittelbehälterabschnitt (138) einschließt, der ausgebildet ist, den Gleitmittelbehälter (146) zu halten.

11. Harnblasenkathetereinführungskit (100) nach einem Anspruch der Ansprüche 1-10, das weiter einen Aufblasbehälter (148) umfasst, der ein Aufblasmittel zum Aufblasen eines Ballons des Harnblasenkatheters (108) einschließt, wobei zumindest ein Abschnitt der vorgeformten Abschnitte des Arbeitstabletts (114, 118, 122) einen Aufblasbehälterabschnitt (138) einschließt, der ausgebildet ist, den Aufblasbehälter (148) zu halten.

12. Harnblasenkathetereinführungskit (100) nach einem Anspruch der Ansprüche 1-11, das weiter ein Genitalvorbereitungskit umfasst, das eine Packung eines Antiseptikums und ein oder mehrere Tupferstäbchen (152) einschließt, wobei zumindest ein Abschnitt der vorgeformten Abschnitte des Arbeitstabletts (114, 118, 122) einen Genitalvorbereitungsabschnitt (140) einschließt, der mit einer Vertiefung (154) ausgebildet ist, um das Antiseptikum aus der Packung zu halten, und mit einem oder mehreren abgewinkelten Kanälen (156), um jeweils die ein oder mehreren Tupferstäbchen (152) mit ihren entsprechenden einen oder mehreren saugfähigen Köpfen (158) in der Vertiefung zu halten.

13. Harnblasenkathetereinführungskit (100) nach Anspruch 12, wobei die Vertiefung (154) bemessen ist, ein Volumen aufzuweisen, das einem gesamten Volumen des Antiseptikums aus der Packung plus einem Volumen der einen oder mehreren saugfähigen Köpfe (158) entspricht, sodass der eine oder die mehreren saugfähigen Köpfe (158) mit dem Antiseptikum gesättigt werden, wenn das Antiseptikum aus der Packung in der Vertiefung (154) abgegeben wird.

14. Harnblasenkathetereinführungskit (100) nach einem Anspruch der Ansprüche 1-13, das weiter einen Urinprobenbehälter umfasst, der in dem Aufbewahrungsraum des Aufbewahrungstabletts (112, 116, 120) angeordnet ist.

15. Harnblasenkathetereinführungskit (100) nach einem Anspruch der Ansprüche 1-14, weiter umfassend:
Schutzkarton, der ausgebildet ist, zumindest die Komponenten des Harnblasenkathetereinführungskits (100) zu schützen, die in dem Arbeitstablett (114, 118, 122) angeordnet sind, wobei das Arbeitstablett (114, 118, 122) eine Lippe um einen Umfang des Arbeitstabletts (114, 118, 122) einschließt, die ausgebildet ist, den Schutzkarton zu halten; und
ein Peri-Care-Kit zwischen dem Schutzkarton und einer Außenverpackung des Harnblasenkathetereinführungskits (100).

## Revendications

1. Kit d'insertion de cathéter urinaire (100) pour une procédure de cathétérisation, comprenant :
un ensemble de plateaux (102, 104, 106) profilé pour un espace entre des jambes partiellement ouvertes d'un patient, l'ensemble de plateaux (102, 104, 106) incluant : un plateau de stockage (112, 116, 120) ; et
un plateau de travail (114, 118, 122) incluant une pluralité de sections préformées tenant une pluralité de composants du kit d'insertion de cathéter urinaire (100), le plateau de travail (114, 118, 122) étant suspendu au-dessus du plateau de stockage (112, 116, 120) dans l'ensemble de plateaux (102, 104, 106), formant ainsi un espace de stockage (128) couvert dans le plateau de stockage (112, 116, 120) ;
un cathéter urinaire (108) disposé dans une section de cathéter (134) des sections préformées ; et
un système de drainage disposé dans l'espace de stockage (128), le système de drainage incluant un réceptacle de drainage et une tubulure de drainage (110) reliée au cathéter urinaire (108),
**caractérisé en ce que**,
les sections préformées du plateau de travail (114, 118, 122) sont agencées conformément à la procédure de cathétérisation, des étapes antérieures dans la procédure de cathétérisation utilisant les composants dans les sections préformées plus proches d'une zone génitale du patient et des étapes ultérieures dans la procédure de cathétérisation utilisant les composants dans les sections préformées plus éloignées de la zone génitale.

2. Kit d'insertion de cathéter urinaire (100) selon la revendication 1, dans lequel l'ensemble de plateaux (102, 104, 106) présente :
une forme de prisme triangulaire,
une forme de prisme trapézoïdal, ou
une forme de goutte d'eau à côtés plats, les côtés plats de la forme de goutte d'eau à côtés plats étant des côtés majeurs de l'ensemble de plateaux (102, 104, 106).

3. Kit d'insertion de cathéter urinaire (100) selon la revendication 1 ou la revendication 2, dans lequel des bords supérieurs du plateau de travail (114, 118, 122) sont chanfreinés ou arrondis pour le confort du patient, et/ou
dans lequel des coins entre les côtés mineurs du plateau de stockage sont arrondis pour le confort du patient.

4. Kit d'insertion de cathéter urinaire (100) selon l'une quelconque des revendications 1 à 3, dans lequel le plateau de travail (114, 118, 122) est en plastique moulé, et/ou dans lequel le plateau de stockage (112, 116, 120) est en carton plié.

5. Kit d'insertion de cathéter urinaire (100) selon l'une quelconque des revendications 1 à 4, comprenant en outre des instructions de cathétérisation étape par étape agencées et marquées sur le plateau de travail (114, 118, 122) conformément à la procédure de cathétérisation, de préférence
dans lequel des étapes des instructions de cathétérisation sont marquées sur le plateau de travail (114, 118, 122) à l'intérieur des sections préformées du plateau de travail (114, 118, 122) ou à côté des sections préformées.

6. Kit d'insertion de cathéter urinaire (100) selon l'une quelconque des revendications 1 à 5, dans lequel le plateau de stockage (112, 116, 120) inclut une ouverture (124) dans une extrémité du plateau de stockage (112, 116, 120) pour faciliter l'accès à l'un quelconque des composants du kit d'insertion de cathéter urinaire (100) stockés dans l'espace de stockage du plateau de stockage (112, 116, 120) tout en travaillant depuis le plateau de travail (114, 118, 122).

7. Kit d'insertion de cathéter urinaire (100) selon l'une quelconque des revendications 1 à 6, dans lequel le plateau de travail (114, 118, 122) inclut une découpe (142) dans une zone d'angle de la section de cathéter (134) formée entre une partie inférieure et une partie d'extrémité de la section de cathéter (134), la tubulure de drainage (110) étant reliée au cathéter urinaire (108) à travers la découpe (142) dans la section de cathéter (134).

8. Kit d'insertion de cathéter urinaire (100) selon la revendication 7, dans lequel la découpe (142) permet à la tubulure de drainage (110) d'être retirée du plateau de stockage (112, 116, 120) sans déconnecter la tubulure de drainage (110) du cathéter urinaire (108).

9. Kit d'insertion de cathéter urinaire (100) selon l'une quelconque des revendications 1 à 8, le système de drainage comprenant en outre un connecteur (144) incluant un port d'échantillonnage d'urine reliant le cathéter urinaire (108) à la tubulure de drainage (110).

10. Kit d'insertion de cathéter urinaire (100) selon l'une quelconque des revendications 1 à 9, comprenant en outre un récipient de lubrifiant (146) incluant du lubrifiant pour le cathéter urinaire (108), au moins une section des sections préformées du plateau de travail (114, 118, 122) incluant une section de récipient de lubrifiant (138) configurée pour maintenir le récipient de lubrifiant (146).

11. Kit d'insertion de cathéter urinaire (100) selon l'une quelconque des revendications 1 à 10, comprenant en outre un récipient d'agent de gonflage (148) incluant un agent de gonflage pour gonfler un ballonnet du cathéter urinaire (108), au moins une section des sections préformées du plateau de travail (114, 118, 122) incluant une section de récipient d'agent de gonflage (138) configurée pour maintenir le récipient d'agent de gonflage (148).

12. Kit d'insertion de cathéter urinaire (100) selon l'une quelconque des revendications 1 à 11, comprenant en outre un kit de préparation génitale incluant un emballage d'un antiseptique et un ou plusieurs bâtonnets écouvillons (152), au moins une section des sections préformées du plateau de travail (114, 118, 122) incluant une section de préparation génitale (140) configurée avec un puits (154) pour maintenir l'antiseptique provenant de l'emballage et un ou plusieurs canaux inclinés (156) pour respectivement maintenir un ou plusieurs bâtonnets écouvillons (152) avec leurs une ou plusieurs têtes absorbantes (158) correspondantes dans le puits.

13. Kit d'insertion de cathéter urinaire (100) selon la revendication 12, dans lequel le puits (154) est dimensionné pour présenter un volume correspondant à un volume entier de l'antiseptique provenant de l'emballage plus un volume des une ou plusieurs têtes absorbantes (158) de sorte que les une ou plusieurs têtes absorbantes (158) deviennent saturées avec l'antiseptique lorsque l'antiseptique provenant de l'emballage est distribué dans le puits (154).

14. Kit d'insertion de cathéter urinaire (100) selon l'une quelconque des revendications 1 à 13, comprenant en outre un récipient d'échantillonnage d'urine disposé dans l'espace de stockage du plateau de stockage (112, 116, 120).

15. Kit d'insertion de cathéter urinaire (100) selon l'une quelconque des revendications 1 à 14, comprenant en outre :
un carton de protection configuré pour protéger au moins les composants du kit d'insertion de cathéter urinaire (100) disposés dans le plateau de travail (114, 118, 122), le plateau de travail (114, 118, 122) incluant une lèvre autour d'un périmètre du plateau de travail (114, 118, 122) configuré pour maintenir le carton de protection ; et
un kit de soins périnéaux entre le carton de protection et un emballage externe du kit d'insertion de cathéter urinaire (100).
